# EUROPEAN PATENT SPECIFICATION

(11) **EP 0 467 703 B1**
(45) Date of publication and mention of the grant of the patent: **24.04.1996**
(21) Application number: 91306601.5
(22) Date of filing: 19.07.1991
(51) Int. Cl.: A61M 25/01, A61M 25/06

(54) **Catheter with needle guard**
Katheter mit Nadelschutz
Cathéter avec protecteur d'aiguille

(30) Priority: 20.07.1990 US 556582
(43) Date of publication of application: 22.01.1992
(73) Proprietor: CRITIKON, INC., Tampa Florida 33634 (US)
(72) Inventor: Ducharme, Leonard C., Tampa, Florida 33624 (US); Chang, Joseph J., Tampa, Florida 33625 (US); Bloom, Richard M., Palm Harbor, Florida 34684 (US); Lemieux, Francis P., Palm Harbor, Florida 34683 (US)
(74) Representative: Fisher, Adrian John

(56) References cited:
- EP-A- 0 343 803
- EP-A- 0 392 765
- US-A- 4 365 630
- US-A- 4 762 516
- US-A- 4 850 961

## Description

This invention relates to intravascular (I.V.) catheters and, in particular, to I.V. catheter assemblies which cover the needle point after use to prevent accidental injury from used needles.

Intravenous catheters for the infusion of fluids into the peripheral veins of a patient are one of the most common devices used in I.V. therapy. I.V. catheters may be produced in two general forms: through-the-needle catheters, in which a catheter is threaded through the needle cannula and into the vein of a patient, and over-the-needle catheters, in which the needle and concentric outer catheter are inserted into the vein and the needle is withdrawn through the emplaced catheter.

A typical over-the-needle I.V. catheter assembly requires the user to remove and then dispose of a contaminated needle after the needle tip and catheter are properly located in a blood vessel of a patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close to where the user is completing work with the catheter, accidental self-inflicted needle injuries are not uncommon. For reasons of the desirability of protecting the user from exposure to blood borne disease such as hepatitis and AIDS, there is an increasing need to protect the user from accidental needle injury.

A catheter design which is directed toward this need is shown in United States Patent number 4,762,516, on which the preamble of claim 1 is based. The catheter shown in this application includes an elongate body which houses a sliding needle guard. In use, the needle with its surrounding catheter tube is inserted through the skin of a patient until the tip of the needle is located in a blood vessel, a position detected by a small flow of blood through the needle and into the flash chamber of the catheter. The user then advances a tab on the top of the needle guard to simultaneously thread the catheter tube into the blood vessel and begin the retraction of the needle from the catheter tube. As the needle is withdrawn from the emplaced catheter, the advance of the tab slides the needle guard out of the housing and along the needle, until the distal end of the guard covers the needle tip and the proximal end of the guard locks in the elongate body. The needle and guard may then be set aside with the needle tip fully protected.

While the arrangement described in this patent application can provide full protection against accidental needle injury, it would be desirable to provide such a catheter in a smaller, smoothly operating configuration which can be readily manipulated by small hands. In accordance with the principles of EP-A-0392765, which forms part of the state of the art by virtue of Article 54(3) EPC, a catheter assembly with needle guard is provided with a semi-tubular needle housing that is open on the upper surface. Located within the housing is a flash chamber with a needle extending from the distal end of the chamber and beyond the distal end of the housing. A tubular needle guard is located for distal movement within the semi-tubular needle housing, and has a distal opening through which the needle extends. The bottom of the needle guard is slotted to fit around the base of the flash chamber. At the rear of the needle guard slot is a portion of a locking mechanism which will engage with and lock in the needle housing when the needle guard is extended to cover the needle.

EP-A-0343803 discloses a catheter assembly which is provided with a protective sheath. The sheath is formed with a pair of claws which engage a pair of ears on the needle housing.

The present invention provides a catheter assembly in accordance with claim 1.

In a preferred embodiment of the present invention the needle guard includes a separate tip piece which enables the mounting of a catheter hub over the needle guard tip. The use of a separate tip also facilitates automated assembly without damage to the sharp pointed needle. When the needle guard is extended, the distal end of the tip piece extends beyond the point of the needle. The needle housing of the preferred embodiment also includes an integral, contoured finger grip located on each side of the needle housing.

In the drawings:
FIGURE 1 is perspective view of a catheter assembly constructed in accordance with the principles of EP-A-0392765, with the sheath in place;
FIGURE 2 is a perspective view of the catheter assembly of FIGURE 1 after removal of the sheath;
FIGURE 3 is a perspective view of the needle housing, needle, and needle guard of the catheter assembly of FIGURE 2 with the catheter removed;
FIGURE 4 is a perspective view of the assembly of FIGURE 3 with the needle guard extended;
FIGURE 5 is a cross-sectional view of the catheter assembly of FIGURE 2;
FIGURES 6a, 6b, 6c, and 6d are views of the needle housing of a catheter assembly;
FIGURES 7a, 7b, and 7c are views of a needle guard suitable for use with the needle housing of FIGURES 6a-6d;
FIGURE 8 is a cross-sectional view of a needle guard tip suitable for use with the needle guard of FIGURES 7a-7c;
FIGURES 9a and 9b are views of a sheath suitable for use with the assembly of FIGURE 1;
FIGURE 10 is a cross-sectional view of the needle housing and needle guard of FIGURES 6a-6d, 7a-7c and 8 with the needle guard retracted;
FIGURE 11 is a cross-sectional view of the needle housing and needle guard of FIGURES 6a-6d, 7a-7c and 8 with the needle guard extended;
FIGURES 12a and 12b are views of the catheter assembly incorporating an alternate configuration of needle guard; and
FIGURES 13a, 13b and 13c are views of the catheter assembly constructed with a secure sheath, in accordance with the present invention.

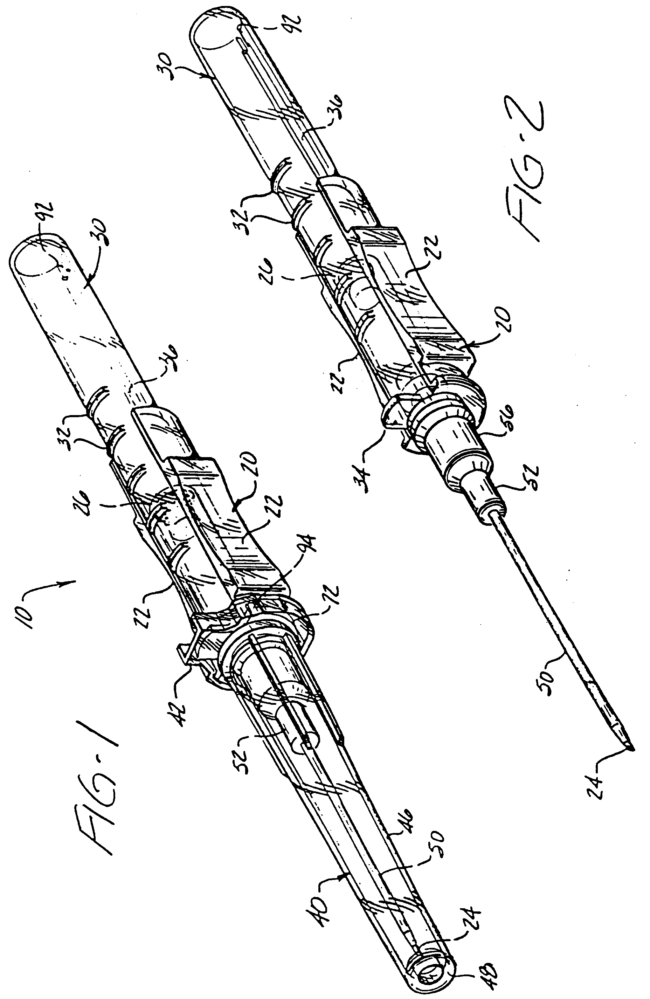

Referring first to Figure 1, a catheter assembly 10 constructed in accordance with the principles of EP-A-0392765 is shown. The assembly 10 includes a needle housing 20 which is semi-tubular in shape and open at the top. Molded on the sides of the needle housing 20 are opposing contoured finger grips 22, one of which is visible in Figure 1. Located inside the semi-tubular needle housing and extending proximally therefrom is a tubular needle guard 30. On the upper surface of the needle guard are a number of small projections 32 which provide surfaces against which a user may press to fully extend the needle guard. These projections permit a user to extend the needle guard with the index or other finger while holding the catheter assembly with one hand. Extending distally from the needle housing 20 is a protective sheath 40 which covers the distally extending needle and catheter.

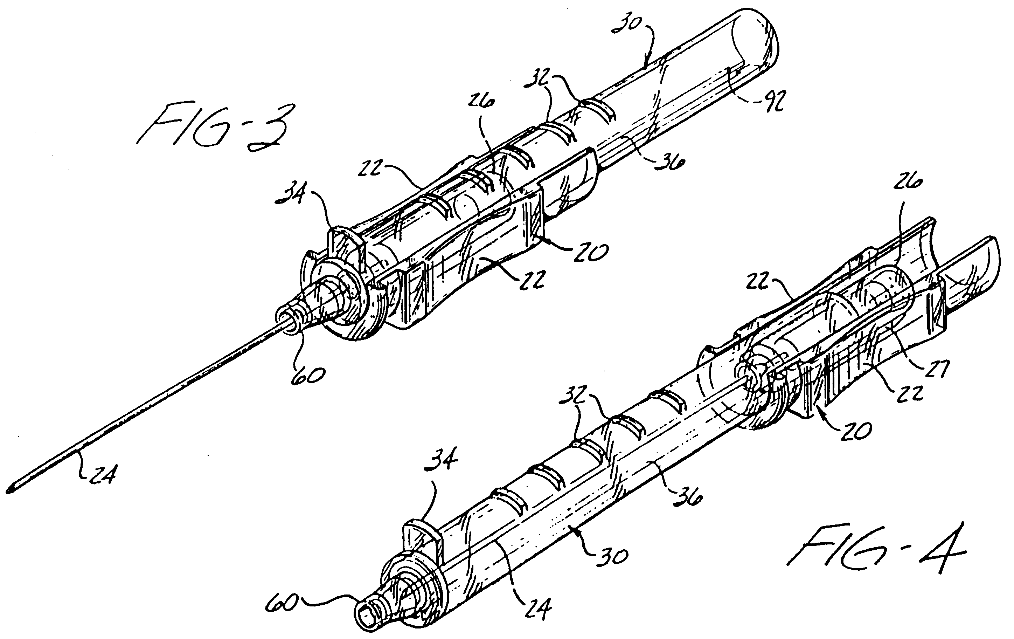

FIGURE 2 illustrates the assembly of FIGURE 1 after removal of the sheath 40. This drawing shows the catheter 50 and its catheter hub 52 mounted on the distal end of the needle guard 30. The point of the needle 24 is seen to extend from the distal tip of the catheter 50. A push-off tab 34 is seen projecting upward from the needle guard proximal the catheter hub 52.

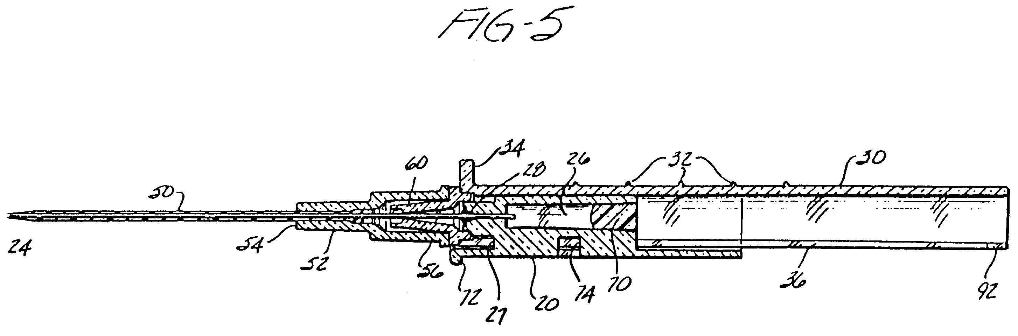

FIGURE 3 shows the assembly of FIGURE 2 prior to mounting the catheter and hub on the distal end of the needle guard. Located on the distal end of the needle guard is a needle guard tip 60, through which the needle 24 extends. FIGURE 4 shows the assembly of FIGURE 3 after the needle guard 30 has been extended to cover the needle 24. In this position the needle guard is locked in its extended position inside the needle housing, and the point of the needle is located inside of the needle guard tip 60.

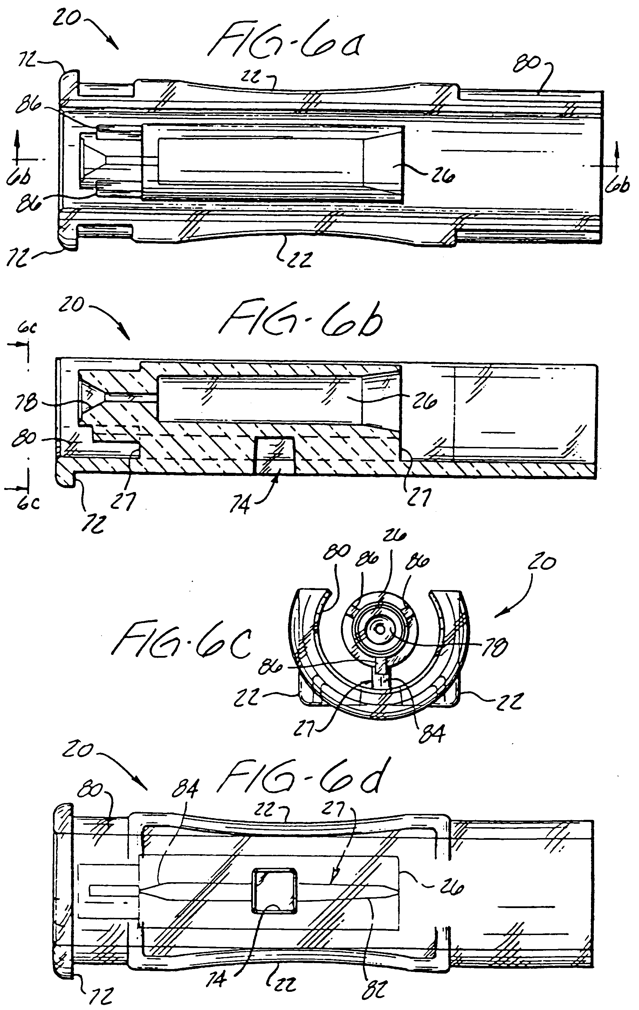

FIGURE 5 is a cross-sectional view of the catheter assembly of FIGURE 2. The catheter 50 is seen to extend from the distal end 54 of the catheter hub 52 and is concentric therewith. The catheter may be attached to its hub by any means known in the art, including adhesively or mechanically by means of a metal eyelet. The larger diameter proximal portion 56 of the catheter hub 52 is flanged at its proximal end for connection to an infusion set, and the inner diameter of the proximal portion of the hub is sized to fit over the distal portion of the needle guard tip 60.

The needle 24 is attached to the distal end of the flash chamber 26 of the needle housing with the proximal end of the needle terminating within the chamber. The needle 24 is affixed in place by adhesive 28. The needle extends through the needle guard tip 60, the needle hub 52, and the catheter 50, with the point of the needle extending from the distal end of the catheter. The rear of the flash chamber 26 is plugged by a microporous plug 70. The needle guard is seen to extend proximal the rear of the needle housing with the needle guard tip 60 affixed to the distal end of the needle guard at the location of the push-off tab 34. The tubular needle guard surrounds the flash chamber 26, with the base 27 of the flash chamber being located in a longitudinal slot 36 at the bottom of the needle guard. As the needle guard slides in the distal direction to cover the needle it is maintained concentric with the needle housing by the concentric tubular construction of the needle housing and needle guard and by the tracking of the base 27 of the flash chamber in the needle guard slot 36.

The needle housing of a catheter assembly is shown in FIGURES 6a, 6b, 6c, and 6d. In the top view of FIGURE 6a the contoured finger grips 22 are seen on either side of the housing. A flange 72 is formed at the distal end of the housing. The flash chamber 26 is seen to be centrally located in the housing. In the cross-sectional view of FIGURE 6b the distal section of the flash chamber to which the needle is attached is seen to extend beyond the base 27 of the flash chamber. The distal opening 78 for the needle 24 is flared, and this flared space is filled with adhesive to attach the needle to the flash chamber. Three ribs 86 are formed uniformly around the distal end of the flash chamber to afford more uniform material flow during the molding process. The proximal opening 76 of the flash chamber is slightly flared to permit ease of insertion of the porous flash plug 70. A rectangular aperture 74 is formed in the base of the housing below the flash chamber 26, and extends upward into the base 27 of the flash chamber.

The distal end view of the needle housing of FIGURE 6c shows the semi-tubular shape of the body 80 of the housing which accommodates mating with the tubular needle guard. The flash chamber 26 is seen in the center of the body extending upward from its base 27. The finger grips 22 are also seen on either side of the housing body.

FIGURE 6d is a bottom plan view of the needle housing, showing the aperture 74 formed in the bottom of the housing. Shown in phantom proximal and distal the aperture is the flash chamber base 27. As the phantom lines 82 indicate, the section of the base 27 proximal the aperture 74 is tapered from a rounded point to a greater width at the aperture 74. As explained below this tapered base and the aperture form a part of the locking mechanism for the needle guard of the catheter assembly. The distal section 84 of the base is also tapered for ease of assembly of the needle guard and housing.

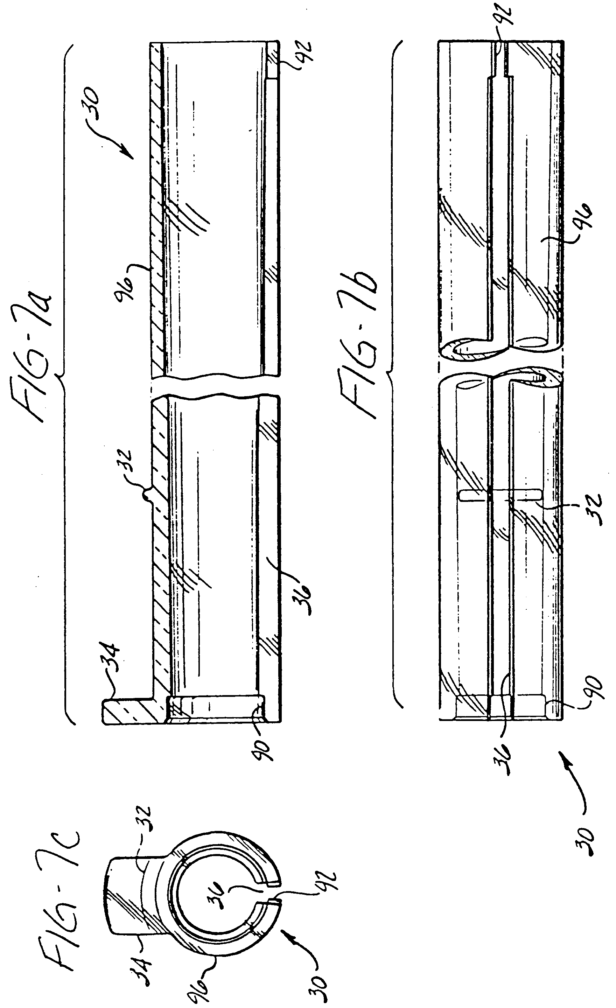

A needle guard suitable for use with the needle housing of FIGURES 6a-6d is shown in FIGURES 7a, 7b, and 7c. FIGURE 7a illustrates the tubular needle guard 30 with its distal push-off tab 34 and upper projections 32. The distal end 90 of the central opening of the tube is formed to accommodate insertion of the needle guard tip 60, to be discussed below. FIGURE 7b is a view of the bottom of the needle guard, with its longitudinal slot 36. The slot 36 is narrowed at its proximal end 92 to form a part of the needle guard locking mechanism. FIGURE 7c is a distal end view of the needle guard 30 which shows the tubular form of the guard. The outer surface 96 of the tubular structure slides smoothly inside the semi-tubular structure 80 of the needle housing of FIGURE 6a-6d.

A needle guard tip 60 suitable for use with the needle guard of FIGURE 7a-7c is shown in cross-section in FIGURE 8. The proximal end 62 of the tip 60 is sized to fit in the distal opening 90 of the needle guard 30. The proximal end of the tip is inserted into the needle guard until the shoulder 63 of the tip contacts the distal end of the guard. The central section 64 of the tip 60 is tapered on both its internal and external surfaces. The distal end 66 of the tip 60 is rounded and open for passage of the needle through the tip.

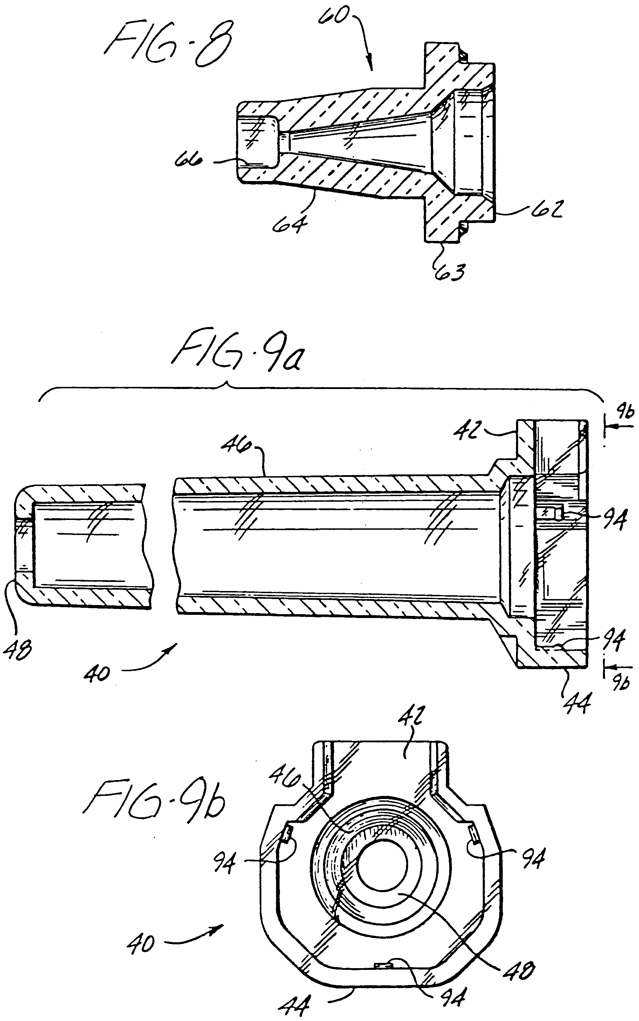

A protective sheath 40 suitable for use with the needle housing of FIGURE 6a-6d is shown in FIGURES 9a and 9b. The sheath releasably attaches to the distal end of the needle housing and is of a sufficient length to cover the catheter and needle. The body 46 of the sheath is slightly tapered from the distal end 48 of the sheath to the flange 44 at the proximal end 44. At the top of the flange 44 is a release tab 42, used to release the sheath from the catheter assembly prior to use of the catheter. The internal diameter of the flange 44 is sized to fit over the distal flange 72 of the needle housing. Three projections 94 are formed in the inner surface of the flange 44 as shown in FIGURE 9b which provide secure connection of the sheath 40 on the housing flange 72.

FIGURE 9b also shows that the outer periphery of the sheath is formed as a series of flat surfaces interconnected by smaller radial surfaces to retard rolling of the catheter assembly.

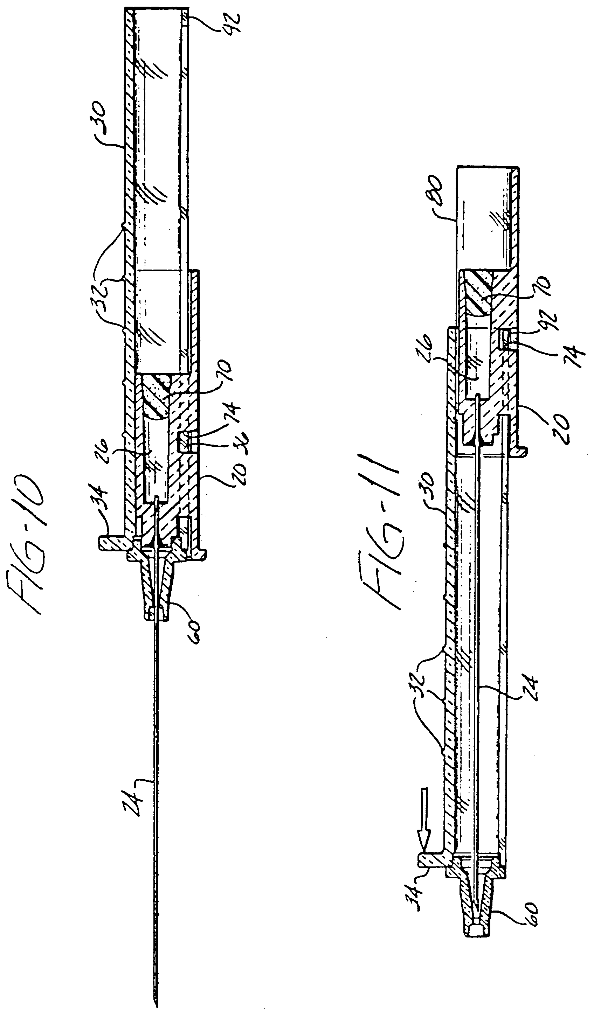

FIGURE 10 shows the subassembly of the needle 24, the needle housing 20, the needle guard 30, the porous flash plug 70, and the needle guard tip 60. Assembly may be accomplished by inserting the flash plug 70 into the proximal end of the flash chamber 26. The needle 24 is inserted into the distal end of the flash chamber and is adhesively secured in place. With the needle and housing oriented vertically, the needle guard 30 is dropped over the needle. The large internal opening of the needle guard minimizes the possibility of contact between the needle guard and the point of the needle, which is important to prevent damage to the sharp needle point during assembly. The needle guard then slides into the needle housing from the distal end of the housing. The tapered distal end 84 of the flash chamber base engages the proximal end of the guard slot 36 to guide the needle guard into the housing around the base 27 of the flash chamber. The guard and housing will slide together until the narrowed proximal end 92 of the slot engages the aperture 74 of the housing, causing the two components to lock together. An instrument is inserted into the aperture 74 and into slot 36 to spread the narrowed portion 92 of the slot and thereby permit the needle guard to proceed fully into the needle housing.

However, the concentric tubular construction of the needle guard and housing also permits the needle guard to slide into the housing from the proximal end of the housing. This is preferable to the distal entry technique described above, for the catheter device can then be assembled without causing the needle guard to pass through its locking position, thereby obviating the need to unlock the narrowed portion 92 of the guard slot during assembly of the device.

With the distal end of the needle guard extending beyond the distal end of the housing, the needle guard tip 60 is dropped over the point of the needle. The small tip can be accurately aligned with its central passageway in line with the needle so that the guard tip can be slipped over the needle without damaging the point of the needle. When the proximal end 62 of the guard tip fully engages the distal opening 90 of the needle guard these two components are ultrasonically welded together. This two-component needle guard thus permits assembly of the catheter device without damage to the needle. The needle guard and tip then slide fully into the needle housing as shown in FIGURE 10. The catheter 50 and hub 52 are then slipped over the needle 24 until the catheter hub 52 is securely seated over the tapered surface 64 of the needle guard tip, as shown in FIGURE 5. The protective sheath may then be slipped over the catheter and needle and snapped onto the needle housing flange 72. The catheter assembly is then packaged for delivery to a user.

The catheter assembly of FIGURE 5 may be used in the conventional manner by inserting the concentric catheter and needle through the skin of a patient and into a blood vessel. When the point of the needle 24 is properly located in the vessel, a small amount of blood will flow through the needle and into the flash chamber 26. Since the needle housing and guard are made of transparent or translucent polymeric materials, the flow of blood will be readily apparent in the flash chamber. The needle is then retracted from the vessel and the catheter 50 threaded into the vessel by grasping the finger grips 22 of the housing with the thumb and fingers and pushing the push-off tab 34 in the distal direction with one finger. This motion will push the catheter hub 52 off of the needle guard tip 60 to advance the catheter. As the needle guard begins to extend out from the distal end of the needle housing such that the push-off tab 34 is beyond the reach of the finger of the user, the user may engage the projections 32 with the finger to continue the distal motion of the needle guard.

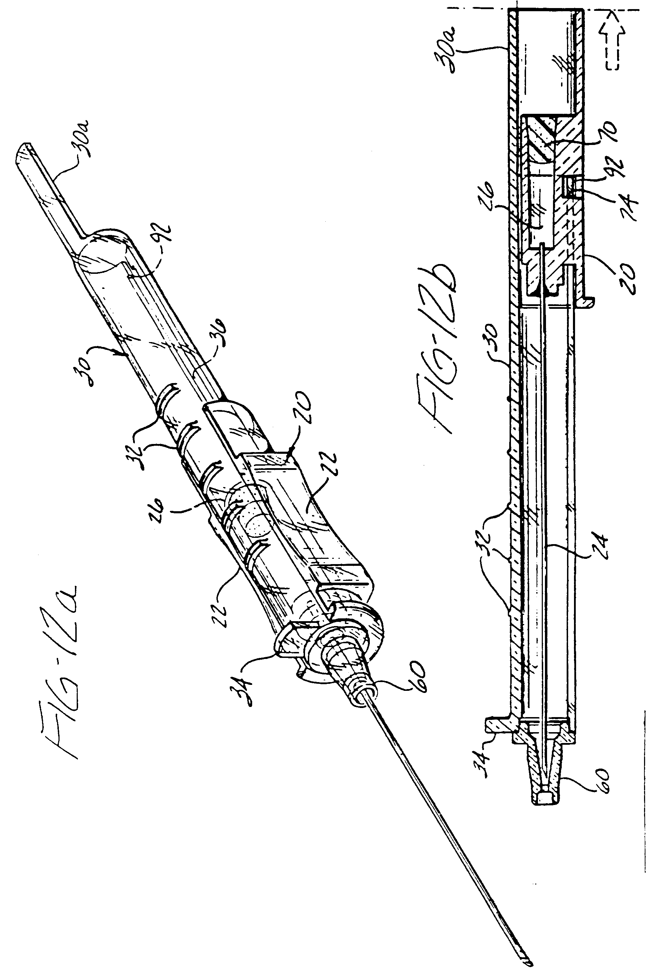

Finally this motion will result in proper threading of the catheter into the vessel and the complete withdrawal of the needle from the patient's body. The needle guard 30 is then advanced to its fullest extension as shown in FIGURE 11. As it does so, the tapered proximal section 82 of the flash chamber base will spread the narrowed proximal portion 92 of the needle guard slot 36 until the narrowed portion 92 finally engages the aperture 74. At the fullest extension of the needle guard from the housing the engagement of the narrowed portion 92 in the aperture 74 will lock the needle guard in its protective position as shown in FIGURE 11. The needle, housing and guard may then be set aside without concern for inadvertent injury to the user or others.

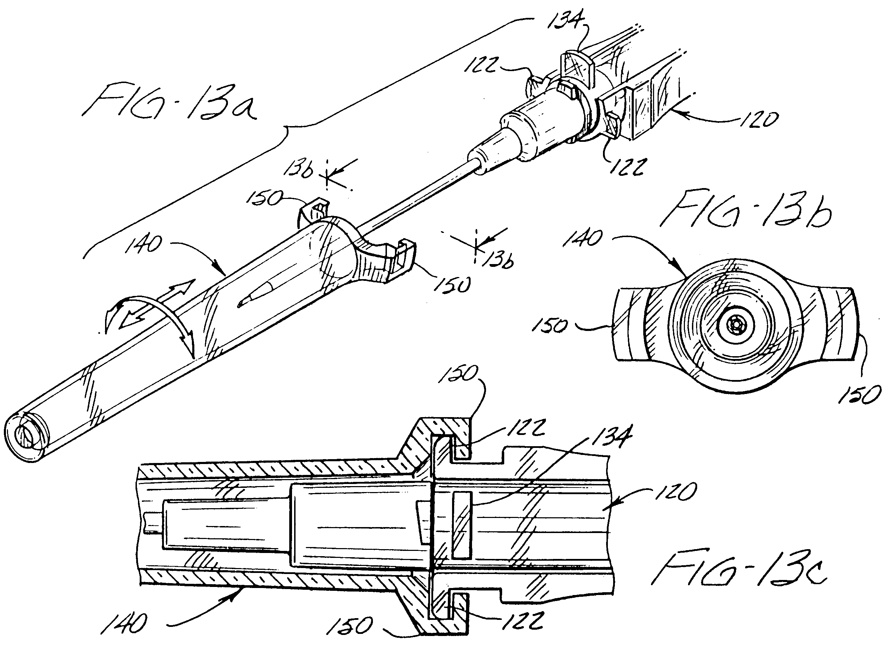

Furthermore, as seen in FIGURES 12a and 12b, an improved needle guard is shown which facilitates operation for the user with smaller hands. Needle guard 30 has attached to it extension 30a forming a finger-like flange at the end of needle guard 30. Preferably this extension 30a is located on the top surface of needle guard 30, so that the structural and positioned integrity of needle guard 30 in relation to housing 20 is maintained. The extra length of extension 30a permits the guard 30 to be advanced and locked into place by the user pressing the back end of the guard against any available surface. The guard 30 is locked into place as in the previous embodiment, but this time with one hand. This design increases the utility of the unit by facilitating and speeding the locking technique.

Figures 13a, 13b and 13c demonstrate the catheter housing attached to a more secure sheath in accordance with the present invention. Here, sheath 140 has two gripping claws 150 as shown in Figures 13a and 13b. Needle housing 120 has two matching flanges 122 to accept the sheath claws 150. Figure 13c is a cross-section view of the housing/sheath junction with the pushoff tab 134 of the needle guard 120 shown. In this design, the sheath 140 can be placed over the housing 120 at 45° rotation to clear the housing flanges 122 and the needle guard tab 134, and then twisted to mate with the flanges 122 to crease a tight locking engagement. The tab 134 protrudes from catheter 110 more than the two housing flanges 122, and therefore serves as a positive stop for the sheath 140 during rotation around housing 120. This assures adequate sheath securement to withstand shocking, tumbling, and vibration. In use, a clinician needs only to twist the sheath 140 either clockwise or counterclockwise and then pull sheath 140 off the housing 120.

## Claims

1. A catheter assembly containing a needle housing (120), a needle guard (30) placed within said housing and movable relative to said housing, a needle (24) formed as part of said housing over which said needle guard is movable to cover said needle, a catheter (50) emplaced over said needle and which remains embedded in the patient when said needle is retracted into said needle guard, and a sheath (140) containing claws (150) and which mates with said housing (120), said sheath covering said exposed needle with said catheter thereon before introduction of said needle and catheter into a patient,
characterised in that said housing (120) is provided with a pair of flanges (122) for engagement with said claws (150), such engagement being effected by rotating said sheath (40) relative to said housing (120).

2. The catheter assembly according to claim 1 wherein said sheath (140) is tubular and extends past said exposed needle (24).

3. A catheter assembly according to claim 1 wherein:
said needle housing (120) is semi-tubular and has an open top;
a flash chamber (26) is located in the interior of said needle housing (120) and within said needle guard (30) and has a hollow needle (24) extending from the distal end thereof;
said needle guard (30) is tubular, is slidably located within said needle housing and includes an aperture at its distal end for passage of said hollow needle therethrough, said needle guard containing an extension outside said semi-tubular housing;
said catheter (50) is attached to a catheter hub assembly suitable for mounting on the distal end of said needle guard; and
means are provided for locking said needle guard (30) in an extended position relative to the distal end of said needle housing.

4. A catheter assembly according to claim 1 wherein:
said needle housing (120) is tubular and has a distal end, a bottom, patient-facing surface and an open top;
said needle (24) is hollow and extends from the distal end of said needle housing;
said needle guard (30) is tubular, and is slidably located within said needle housing and includes at its distal end means for engaging a catheter hub (52), said distal end having an aperture for passage of said hollow needle therethrough, said needle guard having a proximal end containing an extension which causes said proximal end to extend past said housing; and
means are located partially on said needle housing (120) and partially on said needle guard (30) for locking said needle guard in an extended position relative to the distal end of said needle housing;
wherein said needle guard is accessible through said open top of said needle housing for sliding said needle guard distally to its locking position in which the distal end of said hollow needle is located within said needle guard.

5. The catheter assembly of claim 4, wherein said needle housing (120) further comprises a flash chamber (26) located within said housing, wherein said hollow needle (24) extends from said flash chamber and the passageway of said hollow needle is in fluid communication with the interior of said flash chamber.

6. The catheter assembly of claim 5, wherein said flash chamber (26) is located within said slidably mounted needle guard (30).

7. The catheter assembly of claim 6, wherein said flash chamber (26) further includes a base for securing said flash chamber within said needle housing (120), and wherein said needle guard (30) further includes a longitudinally extending slot (36) which engages said flash chamber base (27) as said needle guard slides within said needle housing.

8. The catheter assembly of claim 7, wherein one longitudinal end of said flash chamber base (27) is tapered to engage said needle guard slot (36) during assembly of said needle guard (30) and needle housing (120).

9. The catheter assembly of claim 4, wherein said needle housing (120) further includes two lateral sides, wherein each lateral side includes a contoured finger grip (22).

10. The catheter assembly of claim 4, wherein said needle guard (30) further includes an upward extending push tab (34) located in proximity of the distal end of said needle guard.

11. The catheter assembly of claim 10, wherein said needle guard (30) further includes a plurality of projections (32) located on the upper surface of said needle guard and accessible through said open top of said needle housing (120).

12. The catheter assembly of claim 7, wherein said means for locking includes a narrowed portion (92) of said needle guard slot (36) and an aperture (74) located in said needle housing.

13. The catheter assembly of claim 4, wherein said needle guard (30) comprises a longitudinal tubular section having a relatively large inner diameter and a distally mounted needle guard tip (60) having a relatively small distal opening for passage about said hollow needle (24).

14. The catheter assembly of any of claims 4 to 13, wherein said sheath (140) includes a longitudinally extending, generally planar bottom section.

## Patentansprüche

1. Katheteranordnung, enthaltend ein Nadelgehäuse (120), einen Nadelschutz (30), der innerhalb des Gehäuses angeordnet und relativ zum Gehäuse bewegbar ist, eine Nadel (24), die einen Teil des Gehäuses bildet, über den der Nadelschutz bewegbar ist, um die Nadel abzudecken, einen Katheter (50), der über der Nadel plaziert ist und im Patienten eingebettet bleibt, wenn die Nadel in den Nadelschutz zurückgezogen wird, und eine Klauen (150) enthaltende Hülle (140), die an das Gehäuse (120) angepaßt ist, wobei die Hülle die freiliegende Nadel mit dem darauf angeordneten Katheter vor dem Einführen der Nadel und des Katheters in einen Patienten abdeckt, dadurch gekennzeichnet,
daß das Gehäuse (120) mit einem Paar Flanschen (122) zum Erfassen der Klauen (150) versehen ist, wobei dieses Erfassen durch Drehen der Hülle (40) in bezug auf das Gehäuse (120) bewirkt wird.

2. Katheteranordnung nach Anspruch 1, wobei die Schicht (140) rohrförmig ist und sich über die freiliegende Nadel (24) hinaus erstreckt.

3. Katheteranordnung nach Anspruch 1, wobei:
das Nadelgehäuse (120) halbrohrförmig ist und eine offene Oberseite hat;
eine Entspannungskammer (26) im Innern des Nadelgehäuses (120) und des Nadelschutzes (30) angeordnet ist und eine Hohlnadel (24) aufweist, die sich von dem Vorderende derselben erstreckt;
der Nadelschutz (30) rohrförmig ist und innerhalb des Nadelgehäuses verschiebbar angeordnet ist sowie eine Öffnung an seinem Vorderende für das Hindurchführen der Hohlnadel aufweist, wobei der Nadelschutz eine Verlängerung außerhalb des halbrohrförmigen Gehäuses aufweist;
der Katheter (50) an einer Katheteransatzstückanordnung befestigt ist, die zur Befestigung auf dem Vorderende des Nadelschutzes geeignet ist; und
Mittel zum Verriegeln des Nadelschutzes (30) in einer in bezug auf das Vorderende des Nadelgehäuses ausgefahrenen Stellung vorgesehen sind.

4. Katheteranordnung nach Anspruch 1, wobei:
das Nadelgehäuse (120) rohrförmig ist und ein Vorderende, eine dem Patienten zugekehrte Bodenseite und eine offene Oberseite hat;
die Nadel (24) hohl ist und sich vom Vorderende des Nadelgehäuses erstreckt;
der Nadelschutz (30) rohrförmig ist und innerhalb des Nadelgehäuses verschiebbar angeordnet ist sowie an seinem Vorderende Mittel zum Erfassen eines Katheteransatzstücks (52) aufweist, das Vorderende eine Öffnung für das Hindurchführen der Hohlnadel aufweist, der Nadelschutz ein Hinterende hat, das eine Verlängerung enthält, mittels welcher das Hinterende über das Gehäuse hinaus ausfahrbar ist; und
Mittel teilweise auf dem Nadelgehäuse (120) und teilweise auf dem Nadelschutz (30) zur Verriegelung des Nadelschutzes in einer in bezug auf das Vorderende des Nadelgehäuses ausgefahrenen Position angeordnet sind;
wobei der Nadelschutz durch die offene Oberseite des Nadelgehäuses zur Verschiebung des Nadelschutzes in Richtung seiner vorderen Verriegelungsstellung zugänglich ist, in der das Vorderende der Hohlnadel innerhalb des Nadelschutzes angeordnet ist.

5. Katheteranordnung nach Anspruch 4, wobei das Nadelgehäuse (120) außerdem eine Entspannungskammer (26) umfaßt, die innerhalb des Gehäuses angeordnet ist, wobei die Hohlnadel (24) sich von der Entspannungskammer erstreckt und der Durchgang der Hohlnadel mit dem Innern der Entspannungskammer in Flüssigkeitsverbindung steht.

6. Katheteranordnung nach Anspruch 5, wobei die Entspannungskammer (26) innerhalb des verschiebbar angeordneten Nadelschutzes (30) angeordnet ist.

7. Katheteranordnung nach Anspruch 6, wobei die Entspannungskammer (26) außerdem eine Basis zur Befestigung der Entspannungskammer innerhalb des Nadelgehäuses (120) umfaßt und wobei der Nadelschutz (30) außerdem einen sich längs erstreckenden Schlitz (36) aufweist, der an der Basis (27) der Entspannungskammer anliegt, wenn der Nadelschutz innerhalb des Nadelgehäuses verschoben wird.

8. Katheteranordnung nach Anspruch 7, wobei ein sich längs erstreckendes Ende der Basis (27) der Entspannungskammer verjüngt ist, damit der Nadelschutzschlitz (36) während der Montage des Nadelschutzes (30) und des Nadelgehäuses (120) erfaßbar ist.

9. Katheteranordnung nach Anspruch 4, wobei das Nadelgehäuse (120) weiterhin zwei seitliche Schenkel umfaßt, wobei jeder seitliche Schenkel einen konturierten Fingergriff (22) aufweist.

10. Katheteranordnung nach Anspruch 4, wobei der Nadelschutz (30) ferner eine sich aufwärts erstreckende Stoßzunge (34) umfaßt, die in der Nähe des vorderen Endes des Nadelschutzes angeordnet ist.

11. Katheteranordnung nach Anspruch 10, wobei der Nadelschutz (30) ferner eine Mehrzahl von Vorsprüngen (32) umfaßt, die an der Oberseite des Nadelschutzes angeordnet sind und durch die offene Oberseite des Nadelgehäuses (120) zugänglich sind.

12. Katheteranordnung nach Anspruch 7, wobei die Verriegelungsmittel einen verengten Abschnitt (92) des Nadelschutzschlitzes (36) umfassen und eine Durchbrechung (74) in dem Nadelgehäuse angeordnet ist.

13. Katheteranordnung nach Anspruch 4, wobei der Nadelschutz (30) einen sich längs erstreckenden, rohrförmigen Abschnitt mit einem relativ großen Innendurchmesser und eine vorne angeordnete Nadelschutzspitze (60) mit einer relativ kleinen vorderen Öffnung für das Hindurchführen der Hohlnadel (24) umfaßt.

14. Katheteranordnung nach einem der Ansprüche 4 bis 13, wobei die Hülle (140) einen sich längs erstreckenden, im allgemeinen ebenen Bodenabschnitt umfaßt.

## Revendications

1. Ensemble de cathéter comportant un boîtier d'aiguille (120) une protection d'aiguille (30) placée à l'intérieur dudit boîtier et mobile par rapport audit boîtier, une aiguille (24) formée en tant que partie dudit boîtier sur laquelle ladite protection d'aiguille est mobile pour recouvrir ladite aiguille, un cathéter (50) disposé par dessus ladite aiguille et qui reste enfoncé dans le patient lorsque ladite aiguille est rétractée à l'intérieur de ladite protection d'aiguille, et une gaine (140) comportant des mâchoires (150) et qui est appariée audit boîtier (120), ladite gaine recouvrant ladite aiguille exposée qui comporte ledit cathéter agencé sur celle-ci avant l'introduction de ladite aiguille et du cathéter à l'intérieur d'un patient,
caractérisé en ce que ledit boîtier (120) est muni de deux rebords (122) destinés à venir en prise avec lesdites mâchoires (150), une telle prise étant effectuée par la mise en rotation de ladite gaine (40) par rapport audit boîtier (120).

2. Ensemble de cathéter selon la revendication 1, dans lequel ladite gaine (140) est tubulaire et s étend au-delà de ladite aiguille exposée (24).

3. Ensemble de cathéter selon la revendication 1, dans lequel :
ledit boîtier d'aiguille (120) est semi-tubulaire et comporte une extrémité supérieure ouverte,
une chambre de détente rapide (26) est située à l'intérieur dudit boîtier d'aiguille (120) et à l'intérieur de ladite protection d'aiguille (30) et comporte une aiguille creuse (24) et s'étendant depuis l'extrémité distale de celle-ci,
ladite protection d'aiguille (30) est tubulaire, est située de manière coulissante à l'intérieur dudit boîtier d'aiguille et comporte une ouverture au niveau de son extrémité distale pour le passage de ladite aiguille creuse à travers celle-ci, ladite protection d'aiguille comportant un prolongement vers l'extérieur dudit boîtier semi-tubulaire,
ledit cathéter (50) est fixé à un ensemble de moyeu de cathéter adapté pour être monté sur l'extrémité distale de ladite protection d'aiguille, et
des moyens sont agencés pour verrouiller ladite protection d'aiguille (30) dans une position étendue par rapport à l'extrémité distale dudit boîtier d'aiguille.

4. Ensemble de cathéter selon la revendication 1, dans lequel :
ledit boîtier d'aiguille (120) est tubulaire et a une extrémité distale, une partie inférieure, une surface dirigée vers le patient et une extremité supérieure ouverte,
ladite aiguille (24) est creuse et s'étend à partir de l'extrémité distale dudit boîtier d'aiguille,
ladite protection d'aiguille (30) est tubulaire et est positionnée de manière coulissante à l'intérieur dudit boîtier d'aiguille et comporte au niveau de son extrémité distale des moyens pour venir en prise avec un moyeu de cathéter (52), ladite extrémité distale ayant une ouverture destinée au passage de ladite aiguille creuse à travers celle-ci, ladite protection d'aiguille ayant une extrémité proximale comportant un prolongement qui entraîne ladite extrémité proximale à s'étendre au-delà dudit boîtier, et
des moyens sont positionnés partiellement sur ledit boîtier d'aiguille (120) et partiellement sur ladite protection d'aiguille (30) pour verrouiller ladite protection d'aiguille dans une position étendue par rapport à l'extrémité distale dudit boîtier d'aiguille,
dans lequel ladite protection d'aiguille est accessible à travers ladite partie supérieure ouverte dudit boîtier d'aiguille pour faire coulisser ladite protection d'aiguille de manière distale vers sa position de verrouillage dans laquelle l'extrémité distale de ladite aiguille creuse est située à l'intérieur de ladite protection d'aiguille.

5. Ensemble de cathéter selon la revendication 4, dans lequel ledit boîtier d'aiguille (120) comporte en outre une chambre de détente rapide (26) située à l'intérieur dudit boîtier, dans lequel ladite aiguille creuse (24) s'étend à partir de ladite chambre de détente rapide et le passage de ladite aiguille creuse est en communication de fluide avec la partie intérieure de ladite chambre de détente rapide.

6. Ensemble de cathéter selon la revendication 5, dans lequel ladite chambre de détente rapide (26) est située à l'intérieur de ladite protection d'aiguille montée de manière coulissante (30).

7. Ensemble de cathéter selon la revendication 6, dans lequel ladite chambre de détente rapide (26) comporte en outre une base destinée à la fixation de ladite chambre de détente rapide à l'intérieur dudit boîtier d'aiguille (120), et dans lequel ladite protection d'aiguille (30) comporte en outre une fente s'étendant longitudinalement (36) qui vient en prise avec ladite base (27) de la chambre de détente rapide lorsque ladite protection d'aiguille coulisse à l'intérieur dudit boîtier d'aiguille.

8. Ensemble de cathéter selon la revendication 7, dans lequel une extrémité longitudinale de ladite base (27) de la chambre de détente rapide est conique pour venir en prise avec ladite fente (36) de la protection d'aiguille pendant l'assemblage de ladite protection d'aiguille (30) et du boîtier d'aiguille (120).

9. Ensemble de cathéter selon la revendication 4, dans lequel ledit boîtier d'aiguille (120) comporte en outre deux côtés latéraux, dans lequel chaque côté latéral comporte une saisie profilée pour doigt (22).

10. Ensemble de cathéter selon la revendication 4, dans lequel ladite protection d'aiguille (30) comporte en outre une patte de poussée (34) s'étendant vers le haut située à proximité de l'extrémité distale de ladite protection d'aiguille.

11. Ensemble de cathéter selon la revendication 10, dans lequel ladite protection d'aiguille (30) comporte en outre plusieurs saillies (32) situées sur la surface supérieure de ladite protection d'aiguille et accessibles à travers ladite partie supérieure ouverte dudit boîtier d'aiguille (120).

12. Ensemble de cathéter selon la revendication 7, dans lequel lesdits moyens pour verrouiller comporte une partie rétrécie (92) de ladite fente (36) de la protection d'aiguille et une ouverture (74) située dans ledit boîtier d'aiguille.

13. Ensemble de cathéter selon la revendication 4, dans lequel ladite protection d'aiguille (30) comporte un tronçon longitudinal tubulaire ayant une diamètre intérieur relativement important et un embout (60) de la protection d'aiguille monté distalement ayant une ouverture distale relativement petite destinée au passage autour de ladite aiguille creuse (24).

14. Ensemble de cathéter selon l'une quelconque des revendications 4 à 13, dans lequel ladite gaine (140) comporte un tronçon formant partie intérieure de manière générale plane s'étendant longitudinalement.
